# EUROPEAN PATENT SPECIFICATION

(11) **EP 2 531 601 B1**
(45) Date of publication and mention of the grant of the patent: **16.11.2016**
(21) Application number: 11702021.4
(22) Date of filing: 01.02.2011
(51) Int. Cl.: C12N 15/82, C12N 9/02

(54) **Soybean transformation using HPPD inhibitors as selection agents**
Sojabohnentransformation mittels HPPD-Inhibitoren als auswählbare Wirkstoffe
Transformation de soja au moyen d'inhibiteurs HPPD en tant qu'agents pouvant être sélectionnés

(30) Priority: 01.03.2010 US 339011 P; 02.02.2010 EP 10356007
(43) Date of publication of application: 12.12.2012
(73) Proprietor: Bayer Intellectual Property GmbH, 40789 Monheim (DE)
(72) Inventor: COULOMBIER, Flavie, F-69110 Sainte-Foy-lès-Lyon (FR); ECKERT, Hélène, F-20200 Bastia (FR); FAVRE, Yannick, F-69009 Lyon (FR); PELISSIER, Bernard, F-69370 Saint-Didier-au-Mont-d'Or (FR)
(74) Representative: Guitton, Carole
(86) International application number: PCT/EP2011/051340
(87) International publication number: WO 2011/095460

(56) References cited:
- WO-A1-99/24585
- WO-A2-02/46387
- WO-A2-96/38567
- FR-A1- 2 812 883
- US-A- 5 416 011
- US-B1- 7 473 822

## Description

The invention relates generally to methods for plant transformation and, more particularly, to methods for transforming soybean organogenic cells or tissue using HPPD inhibitors as selection agents. The invention also relates to methods for regenerating transgenic soybean plants from said transformed soybean organogenic cells or tissue, and to transgenic soybean plants and seeds obtained by such methods.

Soybean (soya bean) is one of the most important crops worldwide, providing oil and protein for food and feed purpose. It's the second most important crop in the United States, and is also largely produced in Brazil, Argentina, China and India. Soybean's oil is the most abundant vegetable oil on earth, and the high-protein meal left after oil extract is a very important livestock protein supplement.
Soybeans are one of the crops that have been genetically modified, and genetically modified soybeans are being used in an increasing number of products. In 1997, about 8% of all soybeans cultivated for the commercial market in the United States were genetically modified. In 2009, the figure was 95%.
Despite this increasing demand for genetically modified soybeans, soybean is still a difficult plant to transform and a difficult material for plant regeneration. Improvement and innovation are still needed to obtain a reliable and efficient transformation and regeneration process.

The methods for transforming plant cells generally comprise the following steps:
a) preparing plant cells capable of receiving the heterologous gene,
b) transforming the competent cells with the heterologous gene,
c) growing and selecting the transformed cells comprising the heterologous gene.
   The production of transgenic plants, comprising the heterologous gene integrated into their genome, then consists in carrying out the following steps of:
d) regenerating plants from the transformed, cells and,
e) where appropriate, producing and recovering the seeds of the fertile transformed plants.

As a general rule, and more particularly for plants which are difficult to transform like soybean, the availability of an appropriate and effective mean for selecting the transformed cells is crucial. Furthermore, the selection marker is generally present in the transformed plant, unless subsequent and often heavy means for removing it are applied, and certain type of selectable marker, like genes for resistance to an antibiotic, may be undesirable.

Two major methods are currently used in soybean transformation. The first method utilizes particle bombardment, advantageously of embryogenic calluses, cell cultures on a solid or in suspension, or other proliferative embryogenic tissues (Trick & Finer, 1998; Maughan et al., 1999; Santarem & Finer, 1999; Droste et al., 2002), while the second method involves *Agrobacterium-*mediated transformation of organogenic tissue, such as cotyledonary node tissues (Zhang et al. 1999; Clemente et al., 2000; Olhoft & Somers, 2001; Olhoft et al., 2001), or tissue derived from mature soybean seeds (US 7, 473, 822; EP10356036.3).

McCabe et al. (1988) produced soybean plants using a biolistic-mediated transformation and the *gusA* gene as a reporter, without selection for a resistance marker. The transformed tissues were followed by assessing GUS expression in regenerating shoot and plants. Although this method was used to produce the glyphosate (Raundup®) herbicide-resistant line RR1 that has been used by breeders to produce the commercial Roundup Ready® varieties grown on more than half the total U.S. soybean acres, it was a very labor and cost intensive method, poorly efficient in regard to the number of transformants recovered (Padgette et al. 1995).

Later, more efficient biolistic-mediated methods were developed, using selection for a resistance agent.
In 1991, Finer and McMullen have successfully used embryogenic suspension culture tissue of soybean bombarded with particles coated with plasmid DNAs encoding hygromycin resistance and β-glucuronidase (GUS).
In 1997, hypocotyl explants have been transformed by micro-particle bombardment, using the *bar* gene as selectable marker gene and selection on phosphinothricin (US 5,968,830).
In 2000, the hydroxyphenyl pyruvate dioxygenase (HPPD) gene has been successfully and for the first time used as selectable marker gene for the biolistic-mediated transformation of a proliferative embryogenic tissue, obtained by culturing soybean immature zygotic embryos on a suitable inducer medium (US 6,768,044). HPPD inhibitors act on plant cells by inhibiting the synthesis of plastoquinones and of carotenoids. This action produces a bleaching of the embryogenic tissue which is not harmful to the growth of said cells. Only the transformed plant cells comprising the gene for tolerance to HPPD inhibitors remain green and can be selected since they thus differ from the non-transformed cells. Usually, the selection agent is introduced into the culture medium of the cells after the transformation. In the case of the use of HPPD gene as selectable marker gene for the biolistic-mediated transformation of proliferative embryogenic tissue, the authors have been able to select the transformed cells by introducing the HPPD inhibitor into the culture medium of the competent plant cells before the transformation step, so as to bleach said cells. The bleached competent cells were then transformed, with the gene for tolerance to HPPD inhibitors, as a selection marker, and the transformed cells which have integrated said selection marker into their genome become green, enabling them to be selected (US 6,768,044).
These latest methods have efficiently improved the biolistic-mediated transformation of soybean. Nevertheless, transformation of proliferative embryogenic tissue by particle bombardment has several disadvantages: it requires a prolonged tissue culture period, often yields complex insertion patterns of transgenes into the plant genome, and may result in the regeneration of sterile plants (Liu et al., 1996; Singh et al., 1998; Reddy et al., 2003).
On the contrary, *Agrobacterium*-mediated transformation of organogenic tissue may decrease extensive tissue culture procedures before transformation, and leads to the integration of a simple insertion pattern (generally less than 3 copies, and often a single insertion), which eliminates the risk of subsequent rearrangement between the copies.
For these reasons, having a reproducible and efficient *Agrobacterium*-mediated method, associated with efficient and well-accepted selection means, is highly desirable.

*Agrobacterium*-mediated transformation methods are currently successfully used with organogenic soybean tissues, as the cotyledonary node (US 5,416,011; US 5,959,179) or tissue derived from mature soybean seeds (US 7, 473, 822, EP10356036.3).
Several selection marker genes are currently used for said *Agrobacterium*-mediated transformation methods.
The *nptII* gene which encodes neomycin phosphotransferase for kanamycin resistance has been used successfully (Hinchee et al., 1988; Di et al., 1996; Donaldson and Simmons, 2000; US 5,416,011; US 5,959,179). However, the remaining of an antibiotic resistance marker gene in the final product may be less preferred.
The use of herbicide resistance gene is generally considered as a preferred solution, and two genes have been successfully used for soybean: the *bar* gene that encodes phosphinothricine acetyltransferase which detoxifies the herbicide glufosinate (Zhang et al., 1999; Xing et al., 2000) or bialaphos (Tachibana et al., 1986; Thompson et al., 1987), and the EPSPS (enolpyruvylshikimate-3-phosphate synthase) gene which encodes an enzyme that is resistant to the herbicide glyphosate (US 7,002,058).
Nevertheless, the efficiency rates obtained with the *bar* gene/glufosinate, *bar* gene/bialaphos or EPSPS gene/glyphosate selection are still relatively weak: an efficiency rate of 0 to 3% for transformants derived from cotyledonary nodes has been reported when glufosinate is used as selection agent, while bialaphos gave 0% to 2.1% efficiency (Paz et al., 2004). A similar range of efficiency - between 0.5 and 2 % - has been reported for glyphosate selection (US 7,002,058).
Additionally, a relative long period is needed to select transformed cells using antibiotic or bar selection: around 2 - 3 months, with several transplantings, and it does not avoid getting any false positives.

Described herein is the inventors' surprising discovery that a gene for tolerance to HPPD inhibitors can be used successfully and advantageously as selectable marker gene for *Agrobacterium*-mediated transformation of a soybean organogenic tissue (i.e. soybean organogenic explant). In comparison with the selectable marker genes used so far, the selection, which is a visual one, is really easier, more efficient, and more rapid. This new selection system is indeed associated with an unexpected range of efficiency (up to 4 %), no escape (i.e. no non-transgenic green shoots, no fault positive) and a greatly appreciated gain of time: while around 3 months are necessary with the currently used selection marker genes, only around 3 - 4 weeks are necessary for the selection step using a gene for tolerance to HPPD inhibitors as selectable marker gene.
As an additional advantage, and contrary to the other system including both the antibiotic markers and markers conferring resistance to herbicides other than HPPD inhibitors, the HPPD inhibitors used as selection agents are not lethal for the non-transformed cells. HPPD inhibitors, by inhibiting the synthesis of plastoquinones and of carotenoids, produce the bleaching of the plant cells which is not harmful to the growth of said cells. Only the transformed plant cells comprising the gene for tolerance to HPPD inhibitors remain green and can be visually and easily selected since they thus differ from the white non-transformed, cells. The use of a gene for tolerance to HPPD as selectable marker is therefore a useful system to determine rapidly and easily the efficiency of any new method of transformation/regeneration, as it is possible to dissociate the regeneration efficiency (number of shoots, white or green) from the transformation efficiency (ratio of green shoot/white shoot). In other methods, the number of shoots corresponds to a global transformation and regeneration efficiency, without dissociation between the transformation efficiency from one hand and regeneration efficiency on another hand.

The present invention therefore relates to a method for transforming a soybean plant cell using an *Agrobacterium*-mediated process and a gene or genes for tolerance to HPPD, inhibitors as selection marker gene, said method comprising the steps of:
a) preparing a soybean organogenic explant,
b) exposing said explant to an *Agrobacterium* strain containing a heterologous DNA comprising at least a gene for tolerance to HPPD inhibitors,
c) culturing the explant in the presence of an HPPD inhibitors as selection agent,
d) optionally selecting the transformed plant cell,
**wherein said soybean organogenic explant is a cotyledonous explant, a half-seed explant or a half-embryo-seed explant and wherein the HPPD inhibitor is introduced only after the transformation step b).**

The present invention also relates to a method for preparing a transgenic soybean plant comprising at least a heterologous gene integrated into its genome, comprising a method for transforming a soybean plant cell as described above and further the following steps of:
e) regenerating a plant from the transformed cell.

In a particular embodiment of the invention, step d) (selection of the transformed plant cell) is not performed before the step of regeneration. Under appropriate conditions, shoots are regenerated from cells. These shoots are white when regenerated from an untransformed cell, green when regenerated from a transformed cell. Advantageously, transformed green shoots are then selected based on a visual criteria (green color) and allowed to growth whereas white shoots are eliminated.

Methods other than the visual one may also be used, in replacement or in addition to the visual one. Said methods are well known from the skilled people. One can cite as example PCR (polymerase chain reaction) methods, strip tests,... wherein the target gene can be (one of) the gene(s) conferring the tolerance to HPPD inhibitors or another gene co-introduced with said gene conferring the tolerance to HPPD inhibitors.

### Said methods may also be used in step d)

The present invention therefore relates to a method for preparing a transgenic soybean plant using an *Agrobacterium*-mediated process and a gene or genes for tolerance to HPPD, inhibitors as selection marker gene, said method comprising the steps of:
a) preparing a soybean organogenic explant,
b) exposing said explant to an *Agrobacterium* strain containing a heterologous DNA comprising at least a gene for tolerance to HPPD inhibitors,
c) culturing the explant in the presence of an HPPD inhibitors as selection agent,
d) allowing plants to be regenerated from cells of said explant,
e) selecting transformed plants,
**wherein said soybean organogenic explant is a cotyledonous explant, a half-seed explant or a half-embryo-seed explant and wherein the HPPD inhibitor is introduced only after the transformation step b).**

In a particular embodiment, said transformed plants are selected on visual criteria (green plants).

The hydroxyphenylpyruvate dioxygenases (HPPD; EC 1.13.11.27) are enzymes which catalyse the reaction in which para-hydroxyphenylpyruvate (HPP), a tyrosine degradation product, is transformed into homogentisate (HG), the precursor in plants of tocopherol and plastoquinone (Crouch N.P. et al., 1997; Fritze et al., 2004). Tocopherol acts as a membrane-associated antioxidant. Plastoquinone, firstly acts as an electron carrier between PSII and the cytochrome b6/f complex and secondly, is a redox cofactor for phytoene desaturase, which is involved in the biosynthesis of carotenoids.
Most plants synthesize tyrosine via arrogenate (Abou-Zeid *et al.* 1995; Bonner *et a*/., 1995). In these plants, the HPP is derived only from the degradation of tyrosine. On the other hand, in organisms such as the yeast *Sacharomyces cerevisiae* or the bacterium *Escherichia coli,* it is synthesized by the action of an enzyme, prephenate dehydrogenase (hereinafter referred to as PDH), which converts prephenate to HPP (Lingens *et al.,* 1967; Sampathkumar and Morrisson 1982). In these organisms, the production of HPP is therefore directly connected to the aromatic amino acid biosynthetic pathway (shikimate pathway), and not to the tyrosine degradation pathway.
Several HPPDs and their primary sequences have been described in the state of the art, in particular the HPPDs of bacteria such as *Pseudomonas* (Rüetschi et al., Eur. J. Biochem., 205, 459-466, 1992, WO 96/38567), *Streptomyces avermitilis* (Genebank SAV11864), of fungi such as *Mycosphaerella graminicola* (Genebank AF038152), of plants such as *Arabidopsis* (WO 96/38567, (Genebank AF047834), carrot (WO 96/38567, Genebank 87257), Avena sativa (WO 02/046387), wheat (WO 02/046387), Brachiaria platyphylla (WO 02/046387), Cenchrus echinatus (WO 02/046387), Lolium rigidum (WO 02/046387), Festuca arundinacea (WO 02/046387). Setaria faberi (WO 02/046387), Eleusine indica (WO 02/046387), *Hordeum vulgare* (Genebank HVAJ693), Sorghum (WO 02/046387), Coptis japonica (WO2006132270), Salvia miltiorrhiza (Mol Biol Rep (2009) 36:2019-2029), of *Coccicoides* (Genebank COITRP), of chlamydomonas ES2275365A1, or of mammals such as the mouse *Mus musculus* (Genebank MU54HD) or the pig. By aligning these known sequences, by using the customary means of the art, such as, for example, the method described by Thompson, J.D. et al. (CLUSTAL W: improving the sensitivity of progressive multiple sequence alignment through sequence weighting, positions-specific gap penalties and weight matrix choice. Nucleic Acids Research, 22; 4673-4680, 1994), and accessing these computer programs for sequence alignment which are accessible via the Internet, for example, the skilled person is able to define the sequence homologies in relation to a reference sequence and find the key amino acids or else define common regions.

Inhibition of HPPD leads to uncoupling of photosynthesis, deficiency in accessory light-harvesting pigments and, most importantly, to destruction of chlorophyll by UV-radiation and reactive oxygen species due to the lack of photo protection normally provided by carotenoids (Norris et al. 1995). Photo bleaching of photosynthetically active tissues leads to growth inhibition and plant death.

Some molecules which inhibit HPPD, called HPPD, inhibitors, and which bind specifically to the enzyme in order to inhibit transformation of the HPP into homogentisate, have proven to be very effective selective herbicides.
Most commercially available HPPD inhibitor herbicides belong to one of these four chemical families:
1) the triketones, e.g. sulcotrione [i.e. 2-[2-chloro-4-(methylsulfonyl)benzoyl]-1,3-cyclohexanedione], mesotrione [i.e.2-[4-(methylsulfonyl)-2-nitrobenzoyl]-1,3-cyclohexanedione]; tembotrione [i.e.2-[2-chloro-4-(methylsulfonyl)-3-[(2,2,2,-trifluoroethoxy)methyl] benzoyl]-1,3-cyclo-hexanedionel; tefuryltrione [i.e. 2-[2-chloro-4-(methylsulfonyl)-3-[[(tetrahydro-2-furanyl)methoxy]methyl]benzoyl]-1,3-cyclohexanedione]]; bicyclopyrone [i.e. 4-hydroxy-3-[[2-[(2-methoxyethoxy)methyl]-6-(trifluoromethyl)-3-pyridinyl]carbonyl]bicyclo[3.2.1]oct-3-en-2-one] ; Benzobicyclon [i.e. 3-(2-chloro-4-mesylbenzoyl)-2-phenylthiabicyclo[3.2.1]oct-2-en-4-one]
2) The diketonitriles, e.g. 2-cyano-3-cyclopropyl-1-(2-methylsulphonyl-4-trifluoromethylphenyl)-propane-1,3-dione and 2-cyano-1-[4-(methylsulphonyl)-2-trifluoromethylphenyl]-3-(1-methylcyclopropyl)propane-1,3-fione;
3) the isoxazoles, e.g. isoxaflutole [i.e.(5-cyclopropyl-4-isoxazolyl)[2-(methylsulfonyl)-4-(trifluoromethyl)phenyl]methanonel. In plants, the isoxaflutole is rapidly metabolized in DKN, a diketonitrile compound which exhibits the HPPD inhibitor property; and
4) the pyrazolinates, e.g. topramezone [i.e.[3-(4,5-dihydro-3-isoxazolyl)-2-methyl-4-(methylsulfonyl) phenyl](5-hydroxy-1-methyl-1H-pyrazol-4-yl)methanone], pyrasulfotole [(5-hydraxy-1,3-dimethylpyrazol-4-yl(2-mesyl-4-trifluaromethylphenyl)methanone]; and pyrazofen [2-[4-(2,4-dichlorobenzoyl)-1,3-dimethylpyrazol-5-yloxy]acetophenone].

Tolerance to HPPD inhibitors have been so far obtained via different strategies. One strategy was to overexpress the sensitive enzyme so as to produce quantities of the target enzyme in the plant which are sufficient in relation to the HPPD inhibitor (WO96/38567). Considering such strategy, a gene for tolerance to HPPD inhibitors is a gene encoding a HPPD enzyme, i.e. a HPPD gene. Various HPPD genes have been identified so far, using various sources, including human, bacteria, and plants. As examples, bacteria HPPD genes have been isolated from *Pseudomonas* (EP 0828837), *Sphingomonas elodea* or *Bacillus thuringiensis* (US20050289664). Plant HPPD genes have been isolated from *Arabidopsis thaliana* (EP0877793, EP0938546), the carrot (*Daucus carotta*) (EP 0828837), the oat (*Avena sativa*) (US 7,312,379), the cotton (*Gossypium hirsutum*) (US 7,297,541), the rape (*Brassica napus*), the tomato (*Lycopersicon esculentum*) (US20050289664). A second strategy is to mutate the HPPD in order to obtain a target enzyme which, while retaining its properties of catalysing the transformation or HPP into homogentisate, is less sensitive to HPPD inhibitors than is the native HPPD before mutation. Said strategy, mutated HPPDs satisfying said requirement and genes encoding them, are for example disclosed in EP 1029059, EP patent application N° 08154481.9.

A third strategy is to detoxify the HPPD inhibitors. Plant cytochrome P450s are known to be involved in the metabolism and detoxification of numerous pesticides, and US20090217415 reports the sequence of a cytochrome P450 which confers tolerance to HPPD inhibitors.

A fourth strategy is to by-pass the HPPD-mediatcd production of homogentisate. This has been done by introducing into plant cells a gene encoding an IIPP oxidase, which allows the conversion of HPP to 4-HPA, and genes encoding enzymes allowing the conversion of 4-HPA to homogentisate, wherein all these enzymes are non-sensitive to HPPD inhibitors (EP1330530).
Recently, it has also been shown that the introduction of a Pseudomonas HPPD gene into the plastid genome may confer better tolerance to the HPPD inhibitor isoxaflutol than nuclear transformation (Dufourmantel et al., 2007).
These different strategies may also be combined, as for example combining the HPPD overexpression and the detoxification (WO 2008/150473).

Preferably, the genes for tolerance to HPPD inhibitors comprise, in the direction of transcription, a regulatory promoter sequence which is functional in plant cells and plants, functionally linked to a DNA sequence encoding an HPPD, functionally linked to a regulatory terminator sequence which is functional in plant cells and plants. The sequences encoding HPPDs may be native HPPD sequences, in particular from plants, from microorganisms, from fungi or from mammals, in particular the sequences described in Patent Applications WO 96/38567, US 6 087 563, WO 97/49816 and WO 99/24585 or other references cited in the present application. They are in particular sequences encoding HPPDs from *Pseudomonas fluorescens,* from *Arabidopsis thaliana,* from *Daucus carotta,* from *Avena sativa,* from *Gossypium hirsutum,* from *Brassica napus,* from *Lycopersicon esculentumt*, from wheat, or from Synecocistys. The sequences encoding HPPDs are also sequences mutated, particularly in their C-terminal portion as described in Patent Application WO 99/24585, EP patent application N° 08154481.9 or chimeric HPPDs as described in Patent Application WO 99/24586. According to a preferential embodiment of the invention, the DNA sequence encoding an HPPD is an HPPD sequence mutated in its terminal portion, more particularly a sequence comprising the W336 mutations as described in Patent Application WO 99/24585, EP patent application N° 08154481.9, more preferably the HPPD sequence from *Pseudomonas fluorescens* comprising the W336 mutations as described in Patent Application WO 99/24585, EP patent application N° 08154481.9 and the HPPD sequence from *Arabidopsis thaliana* comprising the W336 mutations as described in EP patent application N° 08154481.9.
According to a particular embodiment of the invention, the gene for tolerance to HPPD inhibitors comprises, in the direction of transcription, a regulatory promoter sequence selected from the promoter of the RuBisCo small subunit from sunflower, described in Patent Application WO 99/25842, or the histone promoter from *Arabidopsis thaliana* combined with the tobacco etch virus (TEV) enhancer as described in Patent Application WO 99/24585, or a CsVMV promoter as described in WO04/053135, functionally linked to a DNA sequence encoding a transit peptide, preferably an optimized transit peptide, as defined hereinafter, functionally linked to a DNA sequence encoding an HPPD as defined above, preferably a sequence encoding an HPPD from *Pseudomonas fluorescens,* comprising the W336 mutation, functionally linked to a regulatory terminator sequence, in particular the NOS terminator sequence.
Particularly suitable examples of genes for tolerance to HPPD are represented by SEQ ID NO 1 and SEQ II) NO 2.
SEQ ID NO 1:
   Promoter : 4541-5257
   Optimized transit peptide: 4130-4487
   HPPDW336 : 3045-4119
   NOS: 2749-3000
SEQ ID NO 2:
   Promoter : 34-1272
   TEV enhancer: 1292-1421
   Optimized transit peptide: 1428-1793
   HPPDW336 : 1795-2869
   NOS: 2914-3165,

Several techniques for transforming soybean by *Agrobacterium,* for preparing suitable explants or tissues, for exposing at least a plant cell capable of receiving the heterologous gene, have been so far disclosed (US5959179, US5416011, US 7,473,822, US 7,002,058, EP 10356036.3).

Preferably, the soybean explant used in the methods of the invention is from an organogenic tissue.
Tissues and cells that are capable of being transformed, whether naturally or artificially, are called competent tissues and competent cells.
For biolistic-mediated transformation methods, competent plant cells may advantageously be from embryogenic calluses, cell cultures on a solid support or in suspension, or other embryogenic proliferative tissues, which are well known to those skilled in the art and widely described in the literature. Advantageously, the competent plant cells are proliferative embryogenic tissues preferably maintained in a semi-solid medium (In Vitro Cell. Dev. Bioll. Plant 35 :451-455, 1999).
For *Agrobacterium*-mediated transformation methods, competent plant cells may be from organogenic tissue. In the meaning of the invention, an organogenic (or organogenetic) tissue is a non-proliferative, differentiated tissue, which is able to develop directly under appropriate conditions, into the organs (stems, leaves, roots,...) of the plants.
An organogenic tissue is therefore different from an embryogenic tissue, which is a proliferative, undifferentiated tissue, which is not able to form any structure such as stem, leaves and roots without being first converted to embryos using an appropriate medium (Finer and Mc Mullen, 1991). After several (3-4) transfers onto this medium, the embryos can be transferred on another medium (Murashige and Skoog medium) when they can develop into the organs of the plants.
In a particular embodiment of the invention, the soybean organogenic explant is a cotyledonous explant prepared from a soybean seedling. Methods comprising preparing a cotyledonous explant from a soybean seedling are disclosed In US5959179 and US5416011. Said methods comprise removing the hypocotyl segment, separating the two cotyledons at the cotyledonary node, and removing the epicotyl. The method advantageously further comprises wounding the explant prior to inoculation by making at least one cut in the petiole region.

In another particular embodiment of the invention, the soybean organogenic explant is a half-seed explant prepared from a mature soybean seed. US 7,473,822 discloses methods comprising imbibing mature soybean seeds, splitting longitudinally the mature soybean seeds and excising totally the embryonic axis, infecting the resulted half-seed explants with *Agrobacterium tumefaciens*, and selecting the transformants.

In another particular embodiment of the invention, the soybean organogenic, explant is a half-embryo-seed explant prepared from a mature soybean seed. EP10356036.3 discloses methods comprising imbibing mature soybean seeds, splitting longitudinally the mature soybean seeds through the embryonic axis, while keeping a piece of embryonic axis on each cotyledon, infecting the resulted half-seed explants with *Agrobacterium tumefaciens,* and selecting the transformants.

An *Agrobacterium*-mediated transformation vector (*Agrobacterium* vector) can be used to insert a heterologous gene into an explant susceptible to infection by *Agrobacterium.* Generally, the gene comprises a promoter, structural coding sequence and a 3' polyadenylation signal or other transcription termination sequence. Promoters which are known or found to cause transcription of gene in plant cells can be used in the present invention. Such promoters may be obtained from plants or viruses and include, but are not necessarily limited to, the 35S and 19S promoters of cauliflower mosaic virus and promoters isolated from plant genes such as EPSPS, ssRUBISCO genes and promoters obtained from T-DNA genes of *Agrobacterium tumefaciens* such as nopaline and mannopine synthases. The particular promoter selected should be capable of causing sufficient expression to result in the desired phenotypic trait. The RNA produced by the gene generally also contains a 5' non-translated leader sequence. This sequence may be derived from any gene and may be specifically modified so as to increase translation of the mRNA. The 5' non-translated regions may be derived from viral RNAs, other suitable eukaryotic genes or a synthetic gene sequence. It may be part of the 5' end of the non-translated region of the structural coding sequence for the encoded polypeptide or derived from an unrelated promoter or coding sequence as discussed above. The 3' non-translated region contains a polyadenylation signal which functions in plants to cause the addition of polyadenylate nucleotides to the 3' end of the mRNA, or other termination sequence. In cases where the structural coding sequence is derived from a plant source one can use the 3' non-translated region naturally associated with the particular plant gene. Examples of other suitable 3' regions are the 3' transcribed, non-translated regions containing the polyadenylation signal of the nopaline synthase (NOS) gene of the *Agrobacterium* tumor-inducing (Ti) plasmid or the conglycinin (7S) storage protein gene.

The invention also relates to a vector sustainable for transforming a soybean plant cell using a *Agrobacterium*-mediated process and comprising at least a heterologous genetic construct comprising at least a gene for tolerance to HPPD inhibitors.

Various *Agrobacterium* strains can be employed, including, but not limited to, *Agrobacterium tumefaciens* and *Agrobacterium rhizogenes.* Preferably, disarmed strains (i.e. for which the tumor- or hair root phenotype inducing genes have been deleted) are utilized. Examples of suitable *A. tumefaciens* strains include strains A208, strain EHA101, LBA4404 (Hood et al., 1986). Examples of suitable *A. rhizogenes* include strain K599, described in US provisional application n°60/606789, US 20090049567. Construction of a disarmed *Agrobacterium* vector is well known in the art, see for example Rogers et al., 1986, Rogers et al., 1987a, Rogers et al., 1987b, and Deblaere et al., 1987.
The explants may be submitted to the infection by *Agrobacterium* in the presence of one or more agents that inhibit browning, such as antioxydants. Examples of said agents include, but are not limited to, cysteine, dithiotreitol, silver nitrate, sodium thiosulfate.

The transformed cells are selected using at least a gene conferring tolerance to HPPD as a selection marker gene, and a HPPD inhibitor as selection agent. The selection marker genes are introduced into the host cells simultaneously with the heterologous gene, either in the same vector, the two genes being associated in a convergent, divergent or colinear manner (WO 95/06128, US 5 731 179), or in two vectors used simultaneously for transforming the plant cells. Under certain conditions (US 5 731 179), and in particular when the heterologous gene and the selection marker gene are introduced separately in two vectors, simultaneously. the heterologous gene encoding a protein of interest and the selection marker gene may integrate on two different chromosomes in the genome of the transformed plant. It is possible, after recovering fertile transformed plants, to eliminate the marker gene in order to produce transformed plants comprising only the heterologous gene encoding a protein of interest. This elimination may take place by self-fertilization or by crossing the transformed plants comprising the heterologous gene and the selection marker gene with a non-transformed variety of the same plant, the segregation of the two genes occurring in conventional Mendelian fashion.

When at least a second heterologous genetic construct is introduced into the soybean plant cell conjointly with the heterologous genetic construct comprising at least a gene for tolerance to HPPD inhibitors, the different heterologous genetic constructs may be included in one or several T-DNA(s). In the case of several T-DNAs, said T-DNAs can be present on one plasmid, or on several plasmids. In the case of several plasmids, said plasmids can be in one Agrobacterium cell or several Agrobacterium cells.

The selection agent, i.e. the HPPD inhibitor, is introduced into the culture medium of the cells after transformation (step c), according to the usual practices of those skilled in the art. HPPD inhibitors act on plant cells by inhibiting the synthesis of plastoquinones and of carotenoids. This action produces a bleaching of the plant cells which is not harmful to the growth of said cells, more particularly in the case of embryogenic tissues. Only the transformed plant cells comprising the gene for tolerance to HPPD inhibitors remain green and can be selected since they thus differ from the non-transformed cells.

Advantageously, the HPPD inhibitors are chosen from isoxazoles (EP 418 175, EP 470 856, EP 487 352, EP 527 036, EP 560 482, EP 682 659, US 5 424 276), in particular isoxaflutole, diketonitriles (DKN) (EP 496 630, EP 496 631), in particular 2-cyano-3-cyclopropyl-1-(2-CH3SO2-4-CF3 phenyl)propan-1,3-dione and 2-cyano-3-cyclopropyl-1-(2-CH3SO2-4-2,3-Cl2 phenyl)propan-1,3-fione, triketones (EP 625 505, EP 625 508, US 5,506,195), in particular sulcotrione, mesotrione, tembotrione, tefuryltrione, bicyclopyrone, and Benzobicyclon and pyrazolinates, in particular topramezone, pyrasulfotole, and pyrazofen.

The suitable amount of HPPD inhibitor introduced into the suitable medium for selecting the transformed cells according to the invention will depend, on the one hand, on the HPPD inhibitor used and, on the other hand, on the explant used. Those skilled in the art will be able to determine this suitable amount using conventional techniques for growing the competent cells at various concentrations of the HPPD inhibitor used.
Preferably, the concentration of HPPD inhibitors is between 0,01 and 50 mg of active material per liter of medium, more preferably between 0,1 and 10 mg/l.
The media and conditions suitable for growing and selecting the transformed cells and the media and conditions for regenerating the transformed plants, arc conventional media well known to those skilled in the art and widely described in the literature, and in particular in the references cited in the present Patent Application. Examples of such media are given in, but not limited in, the examples of the present application

It is understood in the above and in the subsequent text that, when the heterologous gene, the introduction of which into the plant is desired, is a gene for tolerance to HPPD inhibitors, the heterologous gene alone may be introduced and used as the selection marker in the process for transforming the plant cells or the plants.

Preferably, the heterologous genes encoding a protein of interest comprise, in the direction of transcription, a regulatory promoter sequence which is functional in plant cells and plants, functionally linked to a DNA sequence encoding a protein or a peptide of interest, functionally linked to a regulatory terminator sequence which is functional in plant cells and plants.
The DNA sequences encoding a protein or a peptide of interest are generally sequences encoding proteins or peptides which confer, on the transformed plant, novel agronomic properties or improvement of the agronomic quality of the transformed plant.
Among the genes which confer novel agronomic properties on the transformed plants, mention may be made of the DNA sequences encoding proteins which confer tolerance to certain herbicides, those which confer resistance to certain insects, those which confer resistance to nematodes, those which confer tolerance to certain diseases, etc. Such genes are in particular described in Patent Applications WO 91/02071 and WO 95/06128.
Among the DNA sequences encoding proteins which confer tolerance to certain herbicides on the transformed plant cells and plants, mention may be made of the Bar gene which confers tolerance to bialaphos, the gene encoding a suitable EPSPS which confers resistance to herbicides having EPSPS as a target, such as glyphosate and its salts (US 4,535,060, US 4,769,061, US 5,094,945, US 4,940,835, US 5,188,642, US 4,971,908, US 5,145,783, US 5,310,667, US 5,312,910, US 5,627,061, US 5.633.435, FR 2 736 926), the gene encoding glyphosate oxydoreductase (US 5,463,175), or a gene encoding an HPPD which confers tolerance to the herbicides which have HPPD as a target and which are cited above, such as isoxazoles, in particular isoxafutole (FR 95 06800, FR 95 13570), diketonitriles (EP 496 630, EP 496 631) or triketones, in particular tembotrione, sulcotrione or mesotrione (EP 625 505, EP 625 508, US 5,506,195).
Among the DNA sequences encoding a suitable EPSPS which confer resistance to the herbicides which have EPSPS as a target, mention will more particularly be made of the gene which encodes a plant EPSPS, in particular maize EPSPS, which has two mutations, 102 and 106, and which is described in Patent Application FR 2 736 926, hereinafter named EPSPS double mutant, or the gene which encodes an EPSPS isolated from *agrobacterium* and which is described by sequence ID No. 2 and sequence ID No. 3 of US Patent 5,633,435, hereinafter named CP4.
In the cases of the DNA sequences encoding EPSPS or HPPD, and more particularly encoding the genes above, the sequence encoding these enzymes is advantageously preceded by a sequence encoding a transit peptide, in particular encoding the "optimized transit peptide" described in US Patent 5,510,471 or 5,633,448.

Among the DNA sequences encoding proteins of interest which confer novel properties of resistance to insects, mention will more particularly be made of the Bt proteins widely described in the literature and well known to those skilled in the art. Mention will also be made of proteins extracted from bacteria such as Photorhabdus (WO 97/17432 & WO 98/08932).
Among the DNA sequences encoding proteins or peptides of interest which confer novel properties of resistance to diseases, mention will in particular be made of chitinases, glucanases and oxalate oxidase, all these proteins and their coding sequences being widely described in the literature, or antibacterial and/or antifungal peptides, in particular peptides of less than 100 amino acids which are rich in cysteines, such as plant thionins or defensins, and more particularly lytic peptides of any origin comprising one or more disulphide bridges between the cysteines and regions comprising basic amino acids, in particular the following lytic peptides: androctonin (WO 97/30082 and WO 99/09189), drosomycin (WO 99/02717), thanatin (WO 99/24594) or heliomycin (WO 99/53053). According to a particular embodiment of the invention, the protein or peptide of interest is chosen from fungal elicitor peptides, in particular elicitins (Kamoun et al., 1993; Panabières et al., 1995).
Among the DNA sequences encoding proteins or peptides which modify the constitution of the modified plants, mention may be made, in particular, of the DNA sequences encoding proteins or peptides which modify in particular the content and the quality of certain essential fatty acids (EP 666 918) or the content and the quality of the proteins, in particular in the leaves and/or the seeds of said plants. Mention will in particular be made of the genes encoding proteins enriched in sulphur-containing amino acids (Korit, A.A. et al., Eur. J. Biochem. (1991) 195, 329-334 ; WO 98/20133 ; WO 97/41239 ; WO 95/31554 ; WO 94/20828 ; WO 92/14822). Theses proteins enriched in sulphur-containing amino acids will also have the function of trapping and storing excess methionine and/or cysteine, making it possible to avoid the possible problems of toxicity which are linked to an overproduction of these sulphur-containing amino acids, by trapping them. Mention may also be made of the genes encoding peptides rich in sulphur-containing amino acids and more particularly in cysteines, said peptides also having antibacterial and/or antifungal activity. Mention will more particularly be made of plant defensins, as well as lytic peptides of any origin, and more particularly the lytic peptides previously described. Mention will also be made of the SAT proteins described in Patent Applications WO 00/36127, WO 00/04167 and WO 00/01833. As a regulatory sequence which is a promoter in plant cells and plants, use may be made of any promoter sequence of a gene which is naturally expressed in plants, in particular a promoter which is expressed especially in the leaves of plants, such as for example "constitutive'' promoters of bacterial, viral or plant origin, or "light-dependent" promoters, such as that of a plant ribulose-biscarboxylase/oxygenase (RuBisCO) small subunit gene, or any suitable known promoter which may be used. Among the promoters of plant origin, mention will be made of the histone promoters as described in Application EP 0 507 698, or the rice actin promoter (US 5,641,876). Among the promoters of a plant virus gene, mention will be made of that of the cauliflower mosaic virus (CAMV 19S or 35S), or the circovirus promoter (AU 689 311).
Use may also be made of a regulatory promoter sequence specific for particular regions or tissues of plants, and more particularly promoters specific for seeds ([22] Datla, R. et al., Biotechnology Ann. Rev. (1997) 3, 269-296), especially the napin promoter (EP 255 378), the phaseolin promoter, the glutenin promoter, the helianthinin promoter (WO 92/17580), the albumin promoter (WO 98/45460), the oelosin promoter (WO 98/45461), the SAT1 promoter or the SAT3 promoter (PCT/US98/06978, filed on 20 October 1998).

Use may also be made of an inducible promoter advantageously chosen from the phenylalanine ammonia lyase (PAL), HMG-CoA reductase (HMG), chitinase, glucanase, proteinase inhibitor (PI), PR1 family gene, nopaline synthase (nos) and vspB promoters (US 5 670 349, Table 3), the HMG2 promoter (US 5 670 349), the apple beta-galactosidase (ABG1) promoter and the apple aminocyclopropane carboxylate synthase (ACC synthase) promoter (WO 98/45445).
According to the invention, use may also be made, in combination with the promoter, other regulatory sequences, which are located between the promoter and the coding sequence, such as transcription activators ("enhancers"), for instance the translation activator of the tobacco mosaic virus (TMV) described in Application WO 87/07644, or of the tobacco etch virus (TEV) described by Carrington & Freed, for example, or introns such as the adh1 intron of maize or intron 1 of rice actin.
As a regulatory terminator or polyadenylation sequence, use may be made or any corresponding sequence of bacterial origin, such as for example the nos terminator of *Agrobacterium tumefaciens,* of viral origin, such as for example the CaMV 35S terminator, or of plant origin, such as for example a histone terminator as described in Application EP 0 633 317.

The sequences encoding an HPPD, like the sequences encoding a protein or peptide of interest, may comprise functionally linked in 5' or in 3', sequence encoding signals for targeting into various compartments of the plant cell, such as chloroplasts, mitochondria or the vacuole. Such signals are described in the literature and are well known to those skilled in the art. The chloroplast transit peptides may be simple, such as an EPSPS transit peptide (US 5,188,642) or a plant ribulose-biscarboxylase/oxygenase small subunit (RuBisCO ssu) transit peptide, optionally comprising some amino acids of the N-terminal portion of the mature RuBisCO ssu (EP 189 707), or a multiple transit peptide comprising a first plant transit peptide fused to a portion of the N-terminal sequence of a mature protein located in the plastid, fused to a second plant transit peptide as described in Patent EP 508 909, and more particularly the optimized transit peptide comprising a sunflower RuBisCO ssu transit peptide fused to 22 amino acids of the N-terminal end of maize RuBisCO ssu fused to the maize RuBisCO ssu transit peptide as described with its coding sequence in Patent EP 508 909.

In WO2004/024928, it has been shown that the transformation of plants with a gene encoding a PDH enzyme makes it possible to increase the tolerance of said plants to HPPD inhibitors. This increase in tolerance is very significant when the plants transformed with a gene encoding a PDH enzyme are plants that also overexpress an HPPD enzyme. Many genes encoding PDH enzymes are described in the literature, and their sequences can be identified on the website http://www.ncbi.nlm.nih.gov/entrez/. Particularly known is the gene encoding the PDH enzyme of the yeast *Saccharomyces cerevisiae* (Accession No. S46037) as described in Mannhaupt et al. (1989, Gene 85, 303-311), of a bacterium of the *Bacillus* genus, in particular of the species *B*. *subtilis* (Accession No. P20692) as described in Henner et al. (1986, Gene 49 (1) 147-152), of a bacterium of the *Escherichia* genus, in particular of the species *E*. *coli* (Accession No. KMECTD) as described in Hudson et al. (1984, J. Mol. Biol. 180(4). 1023-1051), or of a bacterium of the *Erwinia* genus, in particular of the species E. *herbicola* (Accession No. S29934) as described in Xia et al. (1992, J. Gen. Microbiol. 138(7), 1309-1316).

The invention further relates to methods for transforming a soybean plant cell or for preparing a transgenic soybean plant characterized in that the soybean plant cell or plant which is obtained from a method described above is further transformed, simultaneously or successively, with a gene functional in this plant allowing overexpression of a PDH (prephenate dehydrogenase) enzyme.

The invention further relates to a transgenic soybean plant cell or a soybean plant sustainable to be obtained by a method of the invention.

The invention further relates to the use of a gene or genes conferring the tolerance to HPPD inhibitors as selectable marker gene for the transformation of a soybean organogenic cell or tissue via an *Agrobacterium*-mediated process.
A soybean organogenic plant cell is defined as a plant cell which is part of a soybean organogenic tissue or explant.

### Figures:

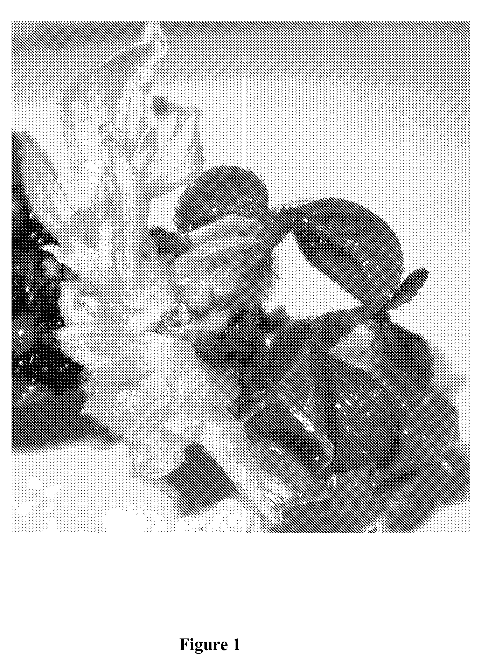

Figure 1: Visual selection with IFT as selectable agent. The untransformed tissue develops into white, elongated shoots, while the transformed material becomes green (dark grey in the white/black photograph) and healthy shoots

The examples hereinafter make it possible to illustrate the invention for the transformation of soybean, without, however, seeking to limit the scope thereof.

All the methods or procedures described below in these examples are given by way of examples and correspond to a choice made from the various methods available in order to attain the same result. Most of the methods for engineering DNA fragments are described in "Current Protocols in Molecular Biology" Volumes 1 and 2, Ausubel F.M. et al., published by Greene Publishing Associates and Wiley-Interscience (1989) or in Molecular cloning, T.Maniatis, E.F.Fritsch, J.Sambrook,1982.

### Example 1 : Cotyledonary node method for transforming soybean

The cotyledonary node method used for transforming soybean has been described by Zhang et al., 1999, Clemente et al. 2000, Xing et al. 2000.

It comprises the steps hereinafter.

### Seed sterilization:

Place seed in an open petri plate as a single layer. Place the petri plate into a standard size desiccator within a fume hood. Place a 250 ml beaker with 100 ml of Chlorox™ bleach in the center of the desiccator. Add 3.3 ml of 12 N HCl, dropwise, along the side of the beaker.

Close the desiccator and let stand overnight.

### Seed germination:

Germination medium is poured in Petri dishes and allowed to solidify. Sterile seeds are placed on the surface of the medium, with the hilum facing downward. Place about 15 seeds per box and incubate 5 days at 24 ° C with a 18/6-Light/Dark photoperiod. After termination, seedlings are placed at 4 ° C for 24h.

### Inoculum preparation:

On the morning before infection, 5mL of YEP + antibiotics are inoculated with a loop of *Agrobacterium tumefaciens,* taken from a fresh spread on LB medium + antibiotic and allowed to grow for 8h at 28°C 200rpm agitation. At night, the subculture is poured into 200ml of YEP + antibiotics. Bacteria are allowed to grow overnight at 28 ° C and 200rpm agitation.

On the day of infection, the *Agrobacterium* culture is centrifugated at 4000rpm, 4 ° C, 15min. the pellet is resuspended in 40 to 50mL of infection medium. The final DO₆₀₀ₙₘ must be between 0.8 and 1. Store on ice.

### Transformation :

Only select seedlings that are green and healthy in appearance. Excise the germinating seed from the root system by making a cut through the hypocotyl region. With a scalpel cut the seed vertically through the hypocotyl region. Remove the embryonic axis tissue that remains attached to the cotyledons.

Make 7-12 slices with a scalpel blade vertical to the axis about the junction between the cotyledon and hypocotyl. Prepare 30-40 explants (an explant is 1 cotyledon with hypocotyl attached, i.e. 2 explants per seed) and initiate inoculations. While the first set of explants are inoculating begin to prepare more explants. Place 25 ml of *Agrobacterium* inoculum into a petri plate. Add the prepared explants. Allow the tissue to sit in the inoculum for 30 minutes, with occasional agitation.

### Cocultivation :

Place explants on co-cultivation plates (5 per plate), adaxial side down (flat).

### Washing:

At the end of coculture, the explants are washed briefly in Wash medium.

### Shoot Induction:

After washing, the explants are placed (5 per plate) on the Shoot Initiation Medium, inclined at 45 °, with the cotyledonary node area imbedded in the medium and upwards. The Shoot Initiation step lasts 1 month (24°C 16/8 photoperiod) with a transfer after 15 days in which the hypocotyl is eliminated to keep only the differentiating area and the cotyledon.

### Shoot elongation:

The cotyledon is removed; explants are cleaned of necrotic tissue. The elongation phase lasts until the appearance of well developed shoots, with transfer every two weeks on fresh Shoot Elongation Medium. (24°C 16/8 photoperiod).

### Rooting or grafting:

Resistant shoots are placed on rooting medium or grafted onto a seedling germinated in vitro (on GM medium).

### Acclimation and greenhouse:

Once roots appeared, plants are placed into soil.

For grafting, wait until the scion is strong to go into the soil.

### MEDIA

- YEP Liquid Medium:
   5 g/L Yeast extract, 10 g/L Peptone, 5 g/L NaCl2. pH to 7.0 with NaOH. Appropriate antibiotics should be added to the medium prior to inoculation.
- Co-cultivation Medium:
   1/10X B5 major salts, 1/10X B5 minor salts, 2.8 mg/L Ferrous, 3.8 mg/L NaEDTA, 30 g/L Sucrose, 3.9 g/L MES (pH 5.4). Filter sterilized 1X B5
   vitamins, GA3 (0.25 mg/L), BAP (1.67 mg/L), Cysteine (400 mg/L), Dithiothrietol (154.2 mg/L), and 40 mg/L acetosyringone are added to this medium after autoclaving.
- Infection Medium:
   1/10X B5 major salts, 1/10X B5 minor salts, 2.8 mg/L Ferrous, 3.8 mg/L NaEDTA, 30 g/L Sucrose, 3.9 g/L MES (pH 5.4). Filter sterilized 1X B5 vitamins, GA3 (0.25 mg/L), BAP (1.67 mg/L), and 40 mg/L acetosyringone are added to this medium after autoclaving.
- Washing Medium:
   1X B5 major salts, 1X B5 minor salts, 28 mg/L Ferrous, 38 mg/L NaEDTA, 30 g/L Sucrose, and 0.59 g/L MES (pH 5.7). Filter sterilized 1X B5 vitamins, BAP (1.11 mg/L), Timentin (100 mg/L), Cefotaxime (200 mg/L), and Vancomycin (50 mg/L) are added to this medium after autoclaving.
- Shoot Induction Medium:
   1X B5 major salts, 1X B5 minor salts, 28 mg/L Ferrous, 38 mg/L NaEDTA, 30 g/L Sucrose, 0.59 g/L MES, and 7 g/L Noble agar (pH 5.7). Filter sterilized 1X B5 vitamins,
   BAP (1.11 mg/L), Timentin (50 mg/L), Cefotaxime (200 mg/L), Vancomycin (50 mg/L) and the selection agent are added to this medium after autoclaving.
- Shoot Elongation Medium:
   1X MS major salts, 1X MS minor salts, 28 mg/L Ferrous, 38 mg/L NaEDTA, 30 g/L Sucrose, 0.59 g/L MES, and 7 g/L Noble agar (pH 5.7). Filter sterilized 1X B5 vitamins, Asparagine (50 mg/L), L-Pyroglutamic Acid (100 mg/L), IAA (0.1 mg/L), GA3 (0.5 mg/L), Zeatin-R (1 mg/L), Timentin (50 mg/L), Cefotaxime (200 mg/L), Vancomycin (50 mg/L), and the selection agent are added to this medium after autoclaving.
- Rooting Medium:
   1X MS major salts, 1X MS minor salts, 28 mg/L Ferrous, 38 mg/L NaEDTA, 20 g/L Sucrose, 0.59 g/L MES, and 7 g/L Noble agar (pH 5.6), Filter sterilized 1X B5 vitamins, Asparagine (50 mg/L), and L-Pyroglutamic Acid (100 mg/L) are added to this medium after autoclaving.

### Example 2: Half-seed explant method for transforming soybean

The half-seed explant method used for transforming soybean has been described by Paz et al., 2006.

It comprises the steps hereinafter.

### Seed sterilization:

Place seed in an open petri plate as a single layer. Place the petri plate into a standard size desiccator within a fume hood. Place a 250 ml beaker with 100 ml of Chlorox™ bleach in the center of the desiccator. Add 3.3 ml of 12 N HCl, dropwise, along the side of the beaker. Close the desiccator and let stand overnight.

### Seed imbibition:

Seeds are placed in Petri dishes and soaked in sterile deionized water for 24 hours prior to inoculation, in the dark, at room temperature.

### Inoculum preparation:

On the morning before infection, 5ml of YEP + antibiotics are inoculated with a loop of *Agrobacterium tumefaciens*, taken from a fresh spread on LB medium + antibiotic and allowed to grow for 8h at 28°C 200rpm agitation. At night, the subculture is poured into 200ml of YEP + antibiotics. Bacteria are allowed to grow overnight at 28 ° C and 200rpm agitation.

On the day of infection, the *Agrobacterium* culture is centrifugated at 4000rpm, 4 ° C, 15min. the pellet is resuspended in 40 to 50ml of infection medium. The final DO₆₀₀ₙₘ must be between 0.8 and 1. Store on ice.

### Transformation:

Soaked seeds are dissected, under sterile conditions, as follow: the seed coat is removed and the two cotyledons are separated. The embryonic axis is totally excised. Each cotyledon is kept as explant for inoculation. About 60 explants are prepared and subsequently inoculated together, for 30 minutes in the *Agrobacterium* inoculum, with occasional agitation.

### Cocultivation :

Place explants on co-cultivation plates (5 to 6 per plate), adaxial (flat) side down. Incubate for 5 days, at 24°C, in a 18:6 photoperiod.

### Washing:

At the end of coculture, the explants are washed briefly in Wash medium.

### Shoot Induction:

After washing, the explants are placed (5 per plate) on the Shoot Initiation Medium, inclined at 45 °, with the cotyledonary node area imbedded in the medium and upwards. The Shoot Initiation step lasts I month (24°C 16/8 photoperiod) with a transfer after 15 days in which the hypocotyl is eliminated to keep only the differentiating area and the cotyledon.

### Shoot elongation:

The cotyledon is removed; explants are cleaned of necrotic tissue. The elongation phase lasts until the appearance of well developed shoots, with transfer every two weeks on fresh Shoot Elongation Medium. (24°C 16/8 photoperiod).

### Rooting or grafting:

Resistant shoots are placed on rooting medium or grafted onto a seedling germinated in vitro (on GM medium).

### Acclimation and greenhouse:

Once roots appeared, plants are placed into soil.

For grafting, wait until the scion is strong to go into the soil.

### MEDIA

- YEP Liquid Medium:
   5 g/L Yeast extract, 10 g/L Peptone, 5 g/L NaCl2. pH to 7.0 with NaOH. Appropriate antibiotics should be added to the medium prior to inoculation.
- Co-cultivation Medium:
   1/10X B5 major salts, 1/10X B5 minor salts, 2.8 mg/L Ferrous, 3.8 mg/L NaEDTA, 30 g/L Sucrose, 3.9 g/L MES (pH 5.4). Filter sterilized 1X B5
   vitamins, GA3 (0.25 mg/L), BAP (1.67 mg/L), Cysteine (400 mg/L), Dithiothrietol (154.2 mg/L), and 40 mg/L acetosyringone are added to this medium after autoclaving.
- Infection Medium:
   1/10X B5 major salts, 1/10X B5 minor salts, 2.8 mg/L Ferrous, 3.8 mg/L NaEDTA, 30 g/L Sucrose, 3.9 g/L MES (pH 5.4). Filter sterilized 1X B5 vitamins, GA3 (0.25 mg/L), BAP (1.67 mg/L), and 40 mg/L acetosyringone are added to this medium after autoclaving.
- Washing Medium:
   1X B5 major salts, 1X B5 minor salts, 28 mg/L Ferrous, 38 mg/L NaEDTA, 30 g/L Sucrose, and 0.59 g/L MES (pH 5.7). Filter sterilized 1X B5 vitamins, BAP (1.11 mg/L), Timentin (100 mg/L), Cefotaxime (200 mg/L), and Vancomycin (50 mg/L) are added to this medium after autoclaving.
- Shoot Induction Medium:
   1X B5 major salts, 1X B5 minor salts, 28 mg/L Ferrous, 38 mg/L NaEDTA, 30 g/L Sucrose, 0.59 g/L MES, and 7 g/L Noble agar (pH 5.7). Filter sterilized 1X B5 vitamins, BAP (1.11 mg/L), Timentin (50 mg/L), Cefotaxime (200 mg/L), Vancomycin (50 mg/L) and the selection agent are added to this medium after autoclaving.
- Shoot Elongation Medium:
   1X MS major salts, 1X MS minor salts, 28 mg/L Ferrous, 38 mg/L NaEDTA, 30 g/L Sucrose, 0.59 g/L MES, and 7 g/L Noble agar (pH 5.7). Filter sterilized 1X B5 vitamins, Asparagine (50 mg/L), L-Pyroglutamic Acid (100 mg/L), IAA (0.1 mg/L), GA3 (0.5 mg/L), Zeatin-R (1 mg/L), Timentin (50 mg/L), Cefotaxime (200 mg/L), Vancomycin (50 mg/L), and the selection agent are added to this medium after autoclaving.
- Rooting Medium:
   1X MS major salts, 1X MS minor salts, 28 mg/L Ferrous, 38 mg/L NaEDTA, 20 g/L Sucrose, 0.59 g/L MES, and 7 g/L Noble agar (pH 5.6). Filter sterilized 1X B5 vitamins, Asparagine (50 mg/L), and L-Pyroglutamic Acid (100 mg/L) are added to this medium after autoclaving.

### Example 3: Half-embryo-seed explant method for transforming soybean

The half-embryo-seed explant method used for transforming soybean has been described in the EP patent application N° 10356036.3. It comprises the steps hereinafter.

Mature Thorne soybean seeds were surface-sterilized for 24h with chlorine gas, in a desicator, in which 5mL HCl 37% were added to 150mL of Domestos in a beaker at the center. Disinfected seeds were then placed in Petri dishes and soaked in sterile deionized water for 24 hours prior to inoculation, in the dark, at room temperature.

Soaked seeds were dissected, under sterile conditions, as follow: Using a #15 scalpel blade, the visible part of the hypocotyl was removed by cutting transversally at the site of emergence. The germinated mature soybean seed was bisected longitudinally precisely through the remaining embryonic axis and the seed coat was removed. It was important at this stage to avoid any tissue breaking during the separation of the cotyledon, and to keep a piece of embryonic axis on each cotyledon. The primary leaves attached to the cotyledon were also removed. Each cotyledon was kept as explant for inoculation.

Cocultivation with *Agrobacterium* strain, regeneration of the plants and medium are conducted as described above.

### Example 4: Construction of the Agrobacterium vectors comprising a gene encoding the HPPD

### a) Construction of pFCO117 (HPPD)

Transformation vector pFCO11 was derived from pSF49, a descendant of pBL150α2 (EP 508909). The bar cassette has first been cloned into pSF49 (NotI/ AvrII), to obtain pFCO20. The cassette contains lox sites for bar removal (cre/lox system) in the event and some meganucleases sites (I-SceI I-CreI, I-CeuI, PI-SceI) for further gene insertion at the same locus by homologous recombination. pFCO20 contains convenient restriction sites for epsps cloning (SbfI/ Swal) and hppd cloning (MscI/ XhoI). Epsps is under the control of Ph4A7, promoter of Arabidopsis thaliana histone H4 gene (Chabouté M, et al., (1987)). The expression of the w336 mutated hppd from *Pseudomonas fluorescens* (Boudec P. et al, (1999); US Patent US6245968) is driven by P35S2, a fragment of the promoter region from the Cauliflower Mosaic Virus 35S transcript, followed by ENtev, an enhancer sequence of tobacco etch virus (Carrington J.C. and Freed D.D. 1990). Hppd proteins are targeted into the chloroplast via the optimized transit peptide TPotpc (Lebrun et al (1996); (US5510471). The TPotpc-hppdPfw336 sequence is codon optimized in order to fit soybean and cotton usage codon.

### b) Construction of pFCO48 (HPPD and PDH)

Transformation vector pFCO48 was derived from pFCO2 The cassette containing the selectable marker hppd and the pdh gene (pFCO45) has been cloned (SmaI/ PacI) in pFCO20 (I-CeuI/ PacI). The expression of the w336 mutated hppd from *Pseudomonas fluorescens* (Boudec P. et al, (1999); US Patent (US6245968) is driven by PssuHa, the light inducible promoter of the small subunit of *Helianthus annuus* Rubisco. The pdh is under the control of Ph4A7 ABBC, a fusion promoter composed out of promotor subunits of *Arabidopsis thaliana* histone H4 gene. Hppd and pdh proteins are targeted into the chloroplast via the optimized transit peptide TPotpc (Lebrun et al (1996); US5510471).

### Example 5: Selection with DKN as selectable agent

Transgenic events (cv Thorne) were generated for construct carrying hppd gene alone (e.i. pFCO117) or in combination with pdh gene (e.i. pFCO48), using DKN as selection agent, as described in exemple 1. Selection agent DKN (diketonitriles) was added in SI (Shoot Induction medium) at a final concentration of 2ppm and for about 4 week. When explants were transferred to SE (Shoot Elongation medium), selection was removed. Under our *in vitro* conditions, DKN does not kill untransformed cells, but still prevents chlorophyll synthesis. The untransformed tissue develops into white, elongated shoots, while the transformed material becomes green and healthily shoots. Hence, DKN provides more for a visual rather than a conventional selection agent. Visual selectable marker dramatically decreases selection time, as untransformed material can be identified and eliminated in as little as 4 weeks after transformation.

### Ref biblio:

- Abou-Zeid et al. 1995, Applied Env Microb 41 : 1298-1302
- Bonner et al., 1995, Plant Cells Physiol. 36,1013-1022
- Clemente et al., 2000, Crop Sci 40: 797-803
- Crouch N.P. et al., 1997, Tetrahedron, 53, 20, 6993-7010
- Deblaere et al., 1987, Methods Enzymol. 153:277-305
- Di et al., 1996, Plant Cell Rep 15: 746-750
- Donaldson and Simmons, 2000, Plant Cell Rep 19: 478-484
- Droste et al., 2002, Euphytica 127:367-376
- Dufourmantel et al., 2007, Plant Biotechnol Journal 5: 118-133
- Finer and McMullen, 1991, In vitro cellular & developmental biology plant, Vol 27 (4): 175-182
- Henner et al.,1986, Gene 49 (1) 147-152
- Hinchee et al., 1988, Bio/Technology 6:915-922
- Hood et al., 1986, J. Bacteriol. 168 :1291-1301
- Hudson et al., 1984, J. Mol. Biol. 180(4), 1023-1051
- Lingens et al., 1967, European J. Biochem 1 : 363-374
- Liu et al., 1996, Plant Cell Tiss Org Cult 47: 33-42
- Mannhaupt et al., 1989, Gene 85, 303-311
- Maughan et al., 1999, In Vitro Cell Dev Biol-Plant 35: 334-349
- McCabe et al., 1988, Bio/Technology 6:923-926
- Olhoft et al., 2001, Plant Cell Rep 20: 731-737
- Olhoft and Somers, 2001, Plant Cell Rep 20: 706-711
- Padgette et al. 1991, J. Biol. Chem., 266, 33
- Paz et al., Euphytica 136: 167-179, 2004
- Paz et al., 2006. Plant Cell Rep. 25: 206.
- Reddy et al., 2003, Plant Cell Rep 21: 676-683
- Rogers et al., 1986, Methods in Enzymology 118: 627-640
- Rogers et al., 1987, Methods in Enzymology 118:627-640.
- Sampathkumar and Morrisson 1982, Bioch Biophys Acta702 : 204-211
- Santarem & Finer, 1999, In Vitro Cell Dev Biol - Plant 35: 451-455
- Singh et al., 1998, Theor Appl Genet 96: 319-324
- Tachibana et al., 1986, J. Pestic Sci 11: 33-37
- Thompson et al., 1987, EMBO J 6: 2519-2523
- Trick & Finer, 1998, Plant Cell Rep 17: 482-488
- Xia et al., 1992, J. Gen. Microbiol. 138(7), 1309-1316
- Xing et al., 2000, In Vitro Cell Dev. Biol. Plant 36:456.
- Zhang et al., 1999, Plant Cell Tiss Org Cult 56: 37-46

## Claims

1. A method for transforming a soybean plant cell using an *Agrobacterium*-mediated process and a gene or genes for tolerance to HPPD inhibitors as selection marker, said method comprising the step of:
a) preparing a soybean organogenic explant,
b) exposing said explant to an *Agrobacterium* strain containing at least a first genetic construct comprising at least a gene for tolerance to an HPPD inhibitor,
c) culturing the explant in the presence of an HPPD inhibitor as selection agent,
d) optionally selecting the transformed plant cells,
wherein said soybean organogenic explant is a cotyledonous explant, a half-seed explant or a half-embryo-seed explant and wherein the HPPD inhibitor is introduced only after the transformation step b).

2. The method according to claim 1 wherein the *Agrobacterium* strain is *Agrobacterium tumefaciens* or *Agrobacterium rhizogenes.*

3. A method for transforming a soybean plant cell according to any one of the claims 1 to 2, **characterized in that** said *Agrobacterium* strain comprises further at least a second heterologous genetic construct which is introduced into said soybean plant cell conjointly with said first heterologous genetic construct comprising at least a gene for tolerance to HPPD inhibitors.

4. The method according to claim 3, **characterized in that** the different heterologous genetic constructs are included in one or several T-DNA(s).

5. The method according to claim 3 or 4 wherein the heterologous genetic constructs comprises a gene conferring resistance to an herbicide, a gene conferring resistance to nematodes, or a gene conferring resistance to insect.

6. A method for preparing a transgenic soybean plant comprising at least a heterologous gene integrated into its genome, comprising the method of any one of claims 1 to 5, supplemented with the following steps of:
e) regenerating plants and seeds comprising said heterolous gene from the transformed cell or transformed tissue.

7. A method for preparing a transgenic soybean plant using an Agrobacterium-mediated process and a gene or genes for tolerance to HPPD inhibitors as selection marker gene, said method comprising the steps of:
a) preparing a soybean organogenic explant,
b) exposing said explant to an *Agrobacterium* strain containing a heterologous DNA comprising at least a gene for tolerance to HPPD inhibitors,
c) culturing the explant in the presence of an HPPD inhibitors as selection agent,
d) allowing plants to be regenerated from cells of said explant,
e) selecting transformed plants,
wherein said soybean organogenic explant is a cotyledonous explant, a half-seed explant or a half-embryo-seed explant and wherein the HPPD inhibitor is introduced only after the transformation step b).

8. The method according to anyone of Claims 1 to 7, **characterized in that** the HPPD inhibitor is chosen from:
- isoxazoles, in particular isoxaflutole;
- diketonitriles, in particular 2-cyano-3-cyclopropyl-1-(2-CH3SO2-4-CF3 phenyl)propan-1,3-dione and 2-cyano-3-cyclopropyl-1-(2-CH3SO2-4-2,3-Cl2 phenyl)propan-1,3-fione;
- triketones, in particular sulcotrione, mesotrione, tembotrione, tefuryltrione, bicyclopyrone, benzobicyclon; and
- pyrazolinates, in particular topramezone, pyrasulfotole or pyrazofen.

9. The method according to any one of claims 1 to 8, **characterized in that** the soybean plant cell or plant is further transformed, simultaneously or successively, with a gene functional in this plant allowing overexpression of a PDH (prephenate dehydrogenase) enzyme.

10. Use of a gene or genes conferring the tolerance to an HPPD inhibitor as selectable marker gene for the transformation of a soybean organogenic cell or tissue via an *Agrobacterium-*mediated process wherein the HPPD inhibitor is introduced only after the transformation step.

## Patentansprüche

1. Verfahren zum Transformieren einer Sojabohnenpflanzenzelle unter Verwendung eines *Agrobacterium*-vermittelten Verfahrens und eines Gens oder von Genen für Toleranz gegenüber HPPD-Inhibitoren als Selektionsmarker, wobei das Verfahren folgenden Schritt umfasst:
a) Herstellen eines organogenen Sojabohnen-Explantats,
b) Aussetzen des Explantats gegenüber einem *Agrobacterium*-Stamm, der mindestens ein erstes genetisches Konstrukt enthält, das mindestens ein Gen für Toleranz gegenüber einem HPPD-Inhibitor umfasst,
c) Kultivieren des Explantats in Gegenwart eines HPPD-Inhibitors als Selektionsmittel,
d) gegebenenfalls Selektieren der transformierten Pflanzenzellen,
wobei das organogene Sojabohnen-Explantat ein Kotyledonen-Explantat, ein Halbsamen-Explantat oder ein Halbembryo-Samen-Explantat ist und wobei der HPPD-Inhibitor erst nach dem Transformationsschritt b) eingebracht wird.

2. Verfahren nach Anspruch 1, wobei es sich bei dem *Agrobacterium-*Stamm um *Agrobacterium tumefaciens* oder *Agrobacterium rhizogenes* handelt.

3. Verfahren zum Transformieren einer Sojabohnenpflanzenzelle nach einem der Ansprüche 1 bis 2, **dadurch gekennzeichnet, dass** der *Agrobacterium-*Stamm ferner mindestens ein zweites heterologes genetisches Konstrukt umfasst, das in die Sojabohnenpflanzenzelle gemeinsam mit dem ersten heterologen genetischen Konstrukt, das mindestens ein Gen für Toleranz gegenüber HPPD-Inhibitoren umfasst, eingebracht wird.

4. Verfahren nach Anspruch 3, **dadurch gekennzeichnet, dass** die verschiedenen heterologen genetischen Konstrukte in einer oder mehreren T-DNA(s) enthalten sind.

5. Verfahren nach Anspruch 3 oder 4, wobei die heterologen genetischen Konstrukte ein Gen, das Resistenz gegenüber einem Herbizid verleiht, ein Gen, das Resistenz gegenüber Nematoden verleiht, ein Gen, das Resistenz gegenüber Insekten verleiht, umfassen.

6. Verfahren zur Herstellung einer transgenen Sojabohnenpflanze, die mindestens ein in ihr Genom integriertes heterologes Gen umfasst, umfassend das Verfahren nach einem der Ansprüche 1 bis 5, das mit den folgenden Schritten ergänzt wird:
e) Regenerieren von Pflanzen und Samen, die das heterologe Gen umfassen, aus der transformierten Zelle oder transformiertem Gewebe.

7. Verfahren zur Herstellung einer transgenen Sojabohnenpflanze unter Verwendung eines *Agrobacterium-*vermittelten Verfahrens und eines Gens oder von Genen für Toleranz gegenüber HPPD-Inhibitoren als Selektionsmarkergen, wobei das Verfahren folgende Schritte umfasst:
a) Herstellen einer organogenen Sojabohnen-Explantats,
b) Aussetzen des Explantats gegenüber einem *Agrobacterium-*Stamm*,* der eine heterologe DNA enthält, die mindestens ein Gen für Toleranz gegenüber HPPD-Inhibitoren umfasst,
c) Kultivieren des Explantats in Gegenwart eines HPPD-Inhibitors als Selektionsmittel,
d) Ermöglichen, dass sich Pflanzen aus Zellen des Explantats regenerieren,
e) Selektieren transformierter Pflanzen,
wobei das organogene Sojabohnen-Explantat ein Kotyledonen-Explantat, ein Halbsamen-Explantat oder ein Halbembryo-Samen-Explantat ist und wobei der HPPD-Inhibitor erst nach dem Transformationsschritt b) eingebracht wird.

8. Verfahren nach einem der Ansprüche 1 bis 7, **dadurch gekennzeichnet, dass** der HPPD-Inhibitor aus Folgenden ausgewählt ist:
- Isoxazolen, insbesondere Isoxaflutol;
- Diketonitrilen, insbesondere 2-Cyano-3-cyclopropyl-1-(2-CH3SO2-4-CF3 phenyl)propan-1,3-dion und 2-Cyano-3-cyclopropyl-1-(2-CH3SO2-4-2,3-Cl2 phenyl)propan-1,3-fion;
- Triketonen, insbesondere Sulcotrion, Mesotrion, Tembotrion, Tefuryltrion, Bicyclopyron, Benzobicyclon; und
- Pyrazolinaten, insbesondere Topramezon, Pyrasulfotol oder Pyrazofen.

9. Verfahren nach einem der Ansprüche 1 bis 8, **dadurch gekennzeichnet, dass** die Sojabohnenpflanzenzelle oder -pflanze gleichzeitig oder aufeinander folgend mit einem in dieser Pflanze funktionellen Gen weiter transformiert wird, das die Überexpression eines PDH-(Prephenatdehydrogenase)-Enzyms ermöglicht.

10. Verwendung eines Gens oder von Genen, das/die Toleranz gegenüber einem HPPD-Inhibitor verleiht/verleihen, als Selektionsmarkergen für die Transformation eine(r/s) organogenen Sojabohnenzelle oder -gewebes über ein *Agrobacterium*-vermitteltes Verfahren, wobei der HPPD-Inhibitor erst nach dem Transformationsschritt eingebracht wird.

## Revendications

1. Méthode de transformation d'une cellule végétale de soja à l'aide d'un procédé utilisant *Agrobacterium* et d'un ou plusieurs gènes de tolérance vis-à-vis d'inhibiteurs d'HPPD comme marqueur de sélection, ladite méthode comprenant les étapes consistant à :
a) préparer un explant organogène de soja,
b) exposer ledit explant à une souche *d'Agrobacterium* contenant au moins une première construction génétique comprenant au moins un gène de tolérance vis-à-vis d'un inhibiteur d'HPPD,
c) cultiver l'explant en présence d'un inhibiteur d'HPPD comme agent de sélection,
d) éventuellement sélectionner les cellules végétales transformées,
dans laquelle ledit explant organogène de soja est un explant cotylédoné, un explant de demi-graine ou un explant de demi-embryon-graine et où l'inhibiteur d'HPPD n'est introduit qu'après l'étape de transformation b).

2. Méthode selon la revendication 1, dans laquelle la souche *d'Agrobacterium* est *Agrobacterium tumefaciens* ou *Agrobacterium rhizogenes.*

3. Méthode de transformation d'une cellule végétale de soja selon l'une quelconque des revendications 1 à 2, **caractérisée en ce que** ladite souche d'*Agrobacterium* comprend en outre au moins une deuxième construction génétique hétérologue qui est introduite dans ladite cellule végétale de soja conjointement avec ladite première construction génétique hétérologue comprenant au moins un gène de tolérance vis-à-vis des inhibiteurs d'HPPD.

4. Méthode selon la revendication 3, **caractérisée en ce que** les différentes constructions génétiques hétérologues sont incluses dans un ou plusieurs ADN-T.

5. Méthode selon la revendication 3 ou 4, dans laquelle les constructions génétiques hétérologues comprennent un gène conférant une résistance vis-à-vis d'un herbicide, un gène conférant une résistance vis-à-vis des nématodes, ou un gène conférant une résistance vis-à-vis des insectes.

6. Méthode de préparation d'une plante de soja transgénique comprenant au moins un gène hétérologue intégré dans son génome, comprenant la méthode selon l'une quelconque des revendications 1 à 5, complémentée par l'étape suivante consistant à :
e) régénérer des plantes et des graines comprenant ledit gène hétérologue à partir de la cellule transformée ou du tissu transformé.

7. Méthode de préparation d'une plante de soja transgénique à l'aide d'un procédé médié par *Agrobacterium* et d'un ou plusieurs gènes de tolérance vis-à-vis d'inhibiteurs d'HPPD comme gène de marqueur de sélection, ladite méthode comprenant les étapes consistant à :
a) préparer un explant organogène de soja,
b) exposer ledit explant à une souche d'*Agrobacterium* contenant un ADN hétérologue comprenant au moins un gène de tolérance vis-à-vis d'inhibiteurs d'HPPD,
c) cultiver l'explant en présence d'un inhibiteur d'HPPD comme agent de sélection,
d) permettre aux plantes de se régénérer à partir des cellules dudit explant,
e) sélectionner les plantes transformées,
dans laquelle ledit explant organogène de soja est un explant cotylédoné, un explant de demi-graine ou un explant de demi-embryon-graine et où l'inhibiteur d'HPPD n'est introduit qu'après l'étape de transformation b).

8. Méthode selon l'une quelconque des revendications 1 à 7, **caractérisée en ce que** l'inhibiteur d'HPPD est choisi parme :
- les isoxazoles, en particulier l'isoxaflutole ;
- les dicétonitriles, en particulier la 2-cyano-3-cyclopropyl-1-(2-CH₃SO₂-4-CF₃-phényl)propan-1,3-dione et la 2-cyano-3-cyclopropyl-1-(2-CH₃SO₂-4-2,3-Cl₂-phényl)-propan-1,3-dione ;
- les tricétones, en particulier la sulcotrione, la mésotrione, la tembotrione, la téfuryltrione, la bicyclopyrone, le benzobicyclon ; et
- les pyrazolinates, en particulier la topramézone, le pyrasulfotole ou le pyrazofen.

9. Méthode selon l'une quelconque des revendications 1 à 8, **caractérisée en ce que** la cellule végétale ou la plante de soja est davantage transformée, de manière simultanée ou successive, par un gène fonctionnel dans cette plante permettant la surexpression d'une enzyme PDH (préphénate déshydrogénase).

10. Utilisation d'un ou plusieurs gènes conférant une tolérance vis-à-vis d'un inhibiteur d'HPPD comme gène marqueur sélectionnable pour la transformation d'une cellule ou d'un tissu organogène de soja via un procédé médié par *Agrobacterium,* dans laquelle l'inhibiteur d'HPPD n'est introduit qu'après l'étape de transformation.


### SEQUENCE LISTING

<110> Bayer CropScience AG
<120> Soybean transformation using HPPD inhibitors as selection agents
<130> BCS 10-4004
<160> 2
<170> PatentIn version 3.5
<210> 1
   <211> 5281
   <212> DNA
   <213> artificial
<220>
   <223> chimeric gene
<220>
   <221> terminator
   <222> (2749)..(3000)
<220>
   <221> gene
   <222> (3045)..(4119)
<220>
   <221> transit_peptide
   <222> (4130)..(4487)
<220>
   <221> promoter
   <222> (4541)..(5257)
<400> 1
   ctagtggcgc cacgcgtgat atcatgcatg ttaacatcga tccatgggcg cgccttaatt    60
   aaatttaaat cagctgcatt aatgaatcgg ccaacgcgcg gggagaggcg gtttgcgtat   120
   tgggcgctct tccgcttcct cgctcactga ctcgctgcgc tcggtcgttc ggctgcggcg   180
   agcggtatca gctcactcaa aggcggtaat acggttatcc acagaatcag gggataacgc   240
   aggaaagaac atgtgagcaa aaggccagca aaaggccagg aaccgtaaaa aggccgcgtt   300
   gctggcgttt ttccataggc tccgcccccc tgacgagcat cacaaaaatc gacgctcaag   360
   tcagaggtgg cgaaacccga caggactata aagataccag gcgtttcccc ctggaagctc   420
   cctcgtgcgc tctcctgttc cgaccctgcc gcttaccgga tacctgtccg cctttctccc   480
   ttcgggaagc gtggcgcttt ctcatagctc acgctgtagg tatctcagtt cggtgtaggt   540
   cgttcgctcc aagctgggct gtgtgcacga accccccgtt cagcccgacc gctgcgcctt   600
   atccggtaac tatcgtcttg agtccaaccc ggtaagacac gacttatcgc cactggcagc   660
   tgccactggt aacaggatta gcagagcgag gtatgtaggc ggtgctacag agttcttgaa   720
   gtggtggcct aactacggct acactagaag gacagtattt ggtatctgcg ctctgctgaa   780
   gccagttacc ttcggaaaaa gagttggtag ctcttgatcc ggcaaacaaa ccaccgctgg   840
   tagcggtggt ttttttgttt gcaagcagca gattacgcgc agaaaaaaag gatctcaaga 900
   agatcctttg atcttttcta cggggtctga cgctcagtgg aacgaaaact cacgttaagg 960
   gattttggtc atgagattat caaaaaggat cttcacctag atccttttaa attaaaaatg 1020
   aagttttaaa tcaatctaaa gtatatatga gtaaacttgg tctgacagtt accaatgctt 1080
   aatcagtgag gcacctatct cagcgatctg tctatttcgt tcatccatag ttgcctgact 1140
   ccccgtcgtg tagataacta cgatacggga gggcttacca tctggcccca gtgctgcaat 1200
   gataccgcga gacccacgct caccggctcc agatttatca gcaataaacc agccagctgg 1260
   aagggccgag cgcagaagtg gtcctgcaac tttatccgcc tccatccagt ctattaattg 1320
   ttgccgggaa gctagagtaa gtagttcgcc agttaatagt ttgcgcaacg ttgttgccat 1380
   tgctacaggc atcgtggtgt cacgctcgtc gtttggtatg gcttcattca gctccggttc 1440
   ccaacgatca aggcgagtta catgatcccc catgttgtgc aaaaaagcgg ttagctcctt 1500
   cggtcctccg atcgttgtca gaagtaagtt ggccgcagtg ttatcactca tggttatggc 1560
   agcactgcat aattctctta ctgtcatgcc atccgtaaga tgcttttctg tgactggtga 1620
   gtactcaacc aagtcattct gagaatagtg tatgcggcga ccgagttgct cttgcccggc 1680
   gtcaatacgg gataataccg cgccacatag cagaacttta aaagtgctca tcattggaaa 1740
   acgttcttcg gggcgaaaac tctcaaggat cttaccgctg ttgagatcca gttcgatgta 1800
   acccactcgt gcacccagct gatcttcagc atcttttact ttcaccagcg tttctgggtg 1860
   agcaaaaaca ggaaggcaaa atgccgcaaa aaagggaata agggcgacac ggaaatgttg 1920
   aatactcata ctcttccttt ttcaatatta ttgaagcatt tatcagggtt attgtctcat 1980
   gagcggatac atatttgaat gtatttagaa aaataaacaa ataggggttc cgcgcacatt 2040
   tccccgaaaa gtgccacctg acgcgccctg tagcggcgca ttaagcgcgg cgggtgtggt 2100
   ggttacgcgc agcgtgaccg ctacacttgc cagcgcccta gcgcccgctc ctttcgcttt 2160
   cttcccttcc tttctcgcca cgttcgccgg ctttccccgt caagctctaa atcgggggct 2220
   ccctttaggg ttccgattta gtgctttacg gcacctcgac cccaaaaaac ttgattaggg 2280
   tgatggttca cgtagtgggc catcgccctg atagacggtt tttcgccctt tgacgttgga 2340
   gtccacgttc tttaatagtg gactcttgtt ccaaactgga acaacactca accctatctc 2400
   ggtctattct tttgatttat aagggatttt gccgatttcg gcctattggt taaaaaatga 2460
   gctgatttaa caaaaattta acgcgaattt taacaaaata ttaacgctta caatttccat 2520
   tcgccattca ggctgcgcaa ctgttgggaa gggcgatcgg tgcgggcctc ttcgctatta 2580
   cgccagctgg gcaactgttg ggaagggcga tcggtgcggg cctcttcgct attacgccag 2640
   ctggcgaaag ggggatgtgc tgcaaggcga ttaagttggg taacgccagg gttttcccag 2700
   tcacgacgtt gtaaaacgac ggccagtgaa ttgcggccgc aattcccgat ctagtaacat 2760
   agatgacacc gcgcgcgata atttatccta gtttgcgcgc tatattttgt tttctatcgc 2820
   gtattaaatg tataattgcg ggactctaat cataaaaacc catctcataa ataacgtcat 2880
   gcattacatg ttaattatta catgcttaac gtaattcaac agaaattata tgataatcat 2940
   cgcaagaccg gcaacaggat tcaatcttaa gaaactttat tgccaaatgt ttgaacgatc 3000
   ggggaaattc gtcgagtcac cctcggccgg gctttttgac gcttaatcgg cggtcaatac 3060
   accacgacgc acctggtcac gttcgatgga ctcgaacagc gccttgaagt tccactcgcc 3120
   aaacccatcg tcgcccttgc gctggatgaa ttcgaagaac accgggccca tcagggtttc 3180
   cgagaagatc tgcagcagca ggcgtttgtc gccttccacg gaagatccgt ccagcaggat 3240
   accgcgtgcc tgcagttgat ccaccggctc gccgtggtca ggcaggcggc cttcgagcat 3300
   ttcgtaataa gtgtctggcg gcgcggtcat gaagcgcatg ccgattttct tcaacgcgtc 3360
   ccaggtcttg accaggtcgt cggtgaggaa cgccacgtgc tggatgcctt cgccgttgaa 3420
   ctgcatcagg aactcttcga tctgccccgc gcccttggac gactcttcgt tcagcgggat 3480
   gcggatcatg ccgtccggcg cactcatggc cttggaagtc aggccggtgt actcgccctt 3540
   gatatcgaag taacgcgctt cacggaagtt gaacaatttc tcgtagaagt tggcccagta 3600
   gaccatgcgg ccgcgataga cgttgtgggt caggtggtcg atgactttga gacctgcacc 3660
   gaccggattg cgctccacac cttcgaggta cacgaagtcg atgtcgtaga tcgagctgcc 3720
   ttcgccgaaa cggtcgatca ggtacaacgg cgcgccgccg atgcccttga tcgccggcag 3780
   gttcaattcc atcggcccgg tgtcaatatg gatcggctgg gcgccgagtt ccagggcgcg 3840
   gttgtaggcc ttttgcgagt ccttcacgcg gaacgccatg ccgcacaccg acgggccgtg 3900
   ttcggccgca aagtaggagg cgatgctgtt gggctcgttg ttgaggatca ggttgatctc 3960
   gccctggcgg tacaggtgca cgttcttgga acggtgggtc gcgactttgg tgaagcccat 4020
   gatctcgaag atcggctcca gggtacccgg cgtcggcgac gcgaattcga tgaattcaaa 4080
   gcccatcagg cccattgggt tttcgtatag atctgccatg caccggatcc ttccgccgtt 4140
   gctgacgttg ccgaggcttc tggaggagcg gcgggcgacg gggaggctgg cggtggactt 4200
   gagcccctgg aacggagcga cggcggtggc cgacgaggcc atcatcacgg tgggcgccat 4260
   tgacagcggc ggcaggtacg acagcgtctc gaacttcttg ttgccgtagg ccggccacac 4320
   ctgcatatat tgaactcttc caccgttgct gggaagggtg gagaagtcgt tagccttctt 4380
   ggtggtgggg aaggcggcgt tggacttaag gccggtgaac ggagccacca tgttggcctg 4440
   agcaggggcg gtccggctaa cggtcgcgac tgaggaggag atcgaagcca tggctgcctg 4500
   gctgcctagt atgtatgtac tcgctgcttg cttgggaatt cgatggtcga gaatccaatg 4560
   agtgacttta gtgattatga gctgtatata taatacttgt acatgagctg cctgccatcc 4620
   aacggataaa aacaaatcta tcttaacttg tagtgattct gagcgtagga tgttgtggct 4680
   cttggaattt catgcatagt gtccacataa tataattgca atttgaagac cttatcatat 4740
   agccaccaga aatggagagc cacgtgtcaa atgcacattg ctcaaaatat cttatctcat      4800
   cttctaaagg agaggtagac atggaagggt cggagggtga gtgtaatttt tatgaatcat      4860
   gaggttaata gtgtgtggtt tatattgtta atgttttaac tatcatgagc gtttgaaaat      4920
   ctgctaccgt aattaagtag cagatgtgtt atttttcatc cacatcccgt cacattgcct      4980
   ataatcaaaa agagtttcaa aaattaccta aaaaccatgt aaattctttg aaacctaccg      5040
   aaattctaaa aagaaaatat tgatatcaaa atacgtgaaa actggaccaa tattacccga      5100
   aactggacca atatgttgta gtgtggttga gccgctattg ataagtagtc tagtgctttt      5160
   aatagtaagg ttggaattat taaagcataa ataaaaaaca aatacaaata caaatttatt      5220
   aagactagaa aaattgtatc atccaagtat tgaattatct agaggatccc cgggggatcc      5280
   a                                                                      5281
<210> 2
   <211> 5909
   <212> DNA
   <213> artificial
<220>
   <223> chimeric gene
<220>
   <221> promoter
   <222> (34)..(1272)
<220>
   <221> enhancer
   <222> (1292)..(1421)
<220>
   <221> transit_peptide
   <222> (1428)..(1793)
<220>
   <221> gene
   <222> (1795)..(2869)
<400> 2
   ggtggcggcc gctctagagc ttgcatgcct gcaggtcgag gagaaatatg agtcgaggca 60
   tggatacact aagttcccct gaagtgagca tgatctttga tgctgagatg attcccagag 120
   caagatagtt tgtgctgcaa gtgacacaat tgtaatgaaa ccaccactca acgaatttac 180
   ttgtggcttt gacatgtcgt gtgctctgtt tgtatttgtg agtgccggtt ggtaattatt 240
   tttgttaatg tgattttaaa acctcttatg taaatagtta ctttatctat tgaagtgtgt 300
   tcttgtggtc tatagtttct caaagggaaa ttaaaatgtt gacatcccat ttacaattga 360
   taacttggta tacacaaact ttgtaaattt ggtgatattt atggtcgaaa gaaggcaata 420
   cccattgtat gttccaatat caatatcaat acgataactt gataatacta acatatgatt 480
   gtcattgttt ttccagtatc aatatacatt aagctactac aaaattagta taaatcacta 540
   tattataaat ctttttcggt tgtaacttgt aattcgtggg tttttaaaat aaaagcatgt      600
   gaaaattttc aaataatgtg atggcgcaat tttattttcc gagttccaaa atattgccgc      660
   ttcattaccc taatttgtgg cgccacatgt aaaacaaaag acgattctta gtggctatca      720
   ctgccatcac gcggatcact aatatgaacc gtcgattaaa acagatcgac ggtttataca      780
   tcattttatt gtacacacgg atcgtatgat tgtcattgtt tttccagtat caatatacat      840
   taagctacta caaaattagt ataaatcact atattataaa tctttttcgg ttgtaacttg      900
   taattcgtgg gtttttaaaa taaaagcatg tgaaaatttt caaataatgt gatggcgcaa      960
   ttttattttc cgagttccaa aatattgccg cttcattacc ctaatttgtg gcgccacatg     1020
   taaaacaaaa gacgattctt agtggctatc actgccatca cgcggatcac taatatgaac     1080
   cgtcgattaa aacagatcga cggtttatac atcattttat tgtacacacg gatcgatatc     1140
   tcagccgtta gatttaatat gcgatctgat tgctcaaaaa atagactctc cgtctttgcc     1200
   tataaaaaca atttcacatc tttctcaccc aaatctactc ttaaccgttc ttcttcttct     1260
   acagacatca atttctctcg actctagaat tcgaaacaca acatatacaa aacaaacgaa     1320
   tctcaagcaa tcaagcattc tacttctatt gcagcaattt aaatcatttc ttttaaagca     1380
   aaagcaattt tctgaaaatt ttcaccattt acgaacgata gccatggctt cgatctcctc     1440
   ctcagtcgcg accgttagcc ggaccgcccc tgctcaggcc aacatggtgg ctccgttcac     1500
   cggccttaag tccaacgccg ccttccccac caccaagaag gctaacgact tctccaccct     1560
   tcccagcaac ggtggaagag ttcaatatat gcaggtgtgg ccggcctacg gcaacaagaa     1620
   gttcgagacg ctgtcgtacc tgccgccgct gtctatggcg cccaccgtga tgatggcctc     1680
   gtcggccacc gccgtcgctc cgttccaggg gctcaagtcc accgccagcc tccccgtcgc     1740
   ccgccgctcc tccagaagcc tcggcaacgt cagcaacggc ggaaggatcc ggtgcatggc     1800
   agatctatac gaaaacccaa tgggcctgat gggctttgaa ttcatcgaat tcgcgtcgcc     1860
   gacgccgggt accctggagc cgatcttcga gatcatgggc ttcaccaaag tcgcgaccca     1920
   ccgttccaag aacgtgcacc tgtaccgcca gggcgagatc aacctgatcc tcaacaacga     1980
   gcccaacagc atcgcctcct actttgcggc cgaacacggc ccgtcggtgt gcggcatggc     2040
   gttccgcgtg aaggactcgc aaaaggccta caaccgcgcc ctggaactcg gcgcccagcc     2100
   gatccatatt gacaccgggc cgatggaatt gaacctgccg gcgatcaagg gcatcggcgg     2160
   cgcgccgttg tacctgatcg accgtttcgg cgaaggcagc tcgatctacg acatcgactt     2220
   cgtgtacctc gaaggtgtgg agcgcaatcc ggtcggtgca ggtctcaaag tcatcgacca     2280
   cctgacccac aacgtctatc gcggccgcat ggtctactgg gccaacttct acgagaaatt     2340
   gttcaacttc cgtgaagcgc gttacttcga tatcaagggc gagtacaccg gcctgacttc     2400
   caaggccatg agtgcgccgg acggcatgat ccgcatcccg ctgaacgaag agtcgtccaa     2460
   gggcgcgggg cagatcgaag agttcctgat gcagttcaac ggcgaaggca tccagcacgt 2520
   ggcgttcctc accgacgacc tggtcaagac ctgggacgcg ttgaagaaaa tcggcatgcg 2580
   cttcatgacc gcgccgccag acacttatta cgaaatgctc gaaggccgcc tgcctgacca 2640
   cggcgagccg gtggatcaac tgcaggcacg cggtatcctg ctggacggat cttccgtgga 2700
   aggcgacaaa cgcctgctgc tgcagatctt ctcggaaacc ctgatgggcc cggtgttctt 2760
   cgaattcatc cagcgcaagg gcgacgatgg gtttggcgag tggaacttca aggcgctgtt 2820
   cgagtccatc gaacgtgacc aggtgcgtcg tggtgtattg accgccgatt aagcgtcaaa 2880
   aagcccggcc gagggtgact cgacgaattt ccccgatcgt tcaaacattt ggcaataaag 2940
   tttcttaaga ttgaatcctg ttgccggtct tgcgatgatt atcatataat ttctgttgaa 3000
   ttacgttaag catgtaataa ttaacatgta atgcatgacg ttatttatga gatgggtttt 3060
   tatgattaga gtcccgcaat tatacattta atacgcgata gaaaacaaaa tatagcgcgc 3120
   aaactaggat aaattatcgc gcgcggtgtc atctatgtta ctagatcggg aattgcggcc 3180
   gcaattcact ggccgtcgtt ttacaacgtc gtgactggga aaaccctggc gttacccaac 3240
   ttaatcgcct tgcagcacat ccccctttcg ccagccagct gcattaatga atcggccaac 3300
   gcgcggggag aggcggtttg cgtattgggc gctcttccgc ttcctcgctc actgactcgc 3360
   tgcgctcggt cgttcggctg cggcgagcgg tatcagctca ctcaaaggcg gtaatacggt 3420
   tatccacaga atcaggggat aacgcaggaa agaacatgtg agcaaaaggc cagcaaaagg 3480
   ccaggaaccg taaaaaggcc gcgttgctgg cgtttttcca taggctccgc ccccctgacg 3540
   agcatcacaa aaatcgacgc tcaagtcaga ggtggcgaaa cccgacagga ctataaagat 3600
   accaggcgtt tccccctgga agctccctcg tgcgctctcc tgttccgacc ctgccgctta 3660
   ccggatacct gtccgccttt ctcccttcgg gaagcgtggc gctttctcat agctcacgct 3720
   gtaggtatct cagttcggtg taggtcgttc gctccaagct gggctgtgtg cacgaacccc 3780
   ccgttcagcc cgaccgctgc gccttatccg gtaactatcg tcttgagtcc aacccggtaa 3840
   gacacgactt atcgccactg gcagctgcca ctggtaacag gattagcaga gcgaggtatg 3900
   taggcggtgc tacagagttc ttgaagtggt ggcctaacta cggctacact agaaggacag 3960
   tatttggtat ctgcgctctg ctgaagccag ttaccttcgg aaaaagagtt ggtagctctt 4020
   gatccggcaa acaaaccacc gctggtagcg gtggtttttt tgtttgcaag cagcagatta 4080
   cgcgcagaaa aaaaggatct caagaagatc ctttgatctt ttctacgggg tctgacgctc 4140
   agtggaacga aaactcacgt taagggattt tggtcatgag attatcaaaa aggatcttca 4200
   cctagatcct tttaaattaa aaatgaagtt ttaaatcaat ctaaagtata tatgagtaaa 4260
   cttggtctga cagttaccaa tgcttaatca gtgaggcacc tatctcagcg atctgtctat 4320
   ttcgttcatc catagttgcc tgactccccg tcgtgtagat aactacgata cgggagggct 4380
   taccatctgg ccccagtgct gcaatgatac cgcgagaccc acgctcaccg gctccagatt 4440
   tatcagcaat aaaccagcca gctggaaggg ccgagcgcag aagtggtcct gcaactttat 4500
   ccgcctccat ccagtctatt aattgttgcc gggaagctag agtaagtagt tcgccagtta 4560
   atagtttgcg caacgttgtt gccattgcta caggcatcgt ggtgtcacgc tcgtcgtttg 4620
   gtatggcttc attcagctcc ggttcccaac gatcaaggcg agttacatga tcccccatgt 4680
   tgtgcaaaaa agcggttagc tccttcggtc ctccgatcgt tgtcagaagt aagttggccg 4740
   cagtgttatc actcatggtt atggcagcac tgcataattc tcttactgtc atgccatccg 4800
   taagatgctt ttctgtgact ggtgagtact caaccaagtc attctgagaa tagtgtatgc 4860
   ggcgaccgag ttgctcttgc ccggcgtcaa tacgggataa taccgcgcca catagcagaa 4920
   ctttaaaagt gctcatcatt ggaaaacgtt cttcggggcg aaaactctca aggatcttac 4980
   cgctgttgag atccagttcg atgtaaccca ctcgtgcacc cagctgatct tcagcatctt 5040
   ttactttcac cagcgtttct gggtgagcaa aaacaggaag gcaaaatgcc gcaaaaaagg 5100
   gaataagggc gacacggaaa tgttgaatac tcatactctt cctttttcaa tattattgaa 5160
   gcatttatca gggttattgt ctcatgagcg gatacatatt tgaatgtatt tagaaaaata 5220
   aacaaatagg ggttccgcgc acatttcccc gaaaagtgcc acctgacgcg ccctgtagcg 5280
   gcgcattaag cgcggcgggt gtggtggtta cgcgcagcgt gaccgctaca cttgccagcg 5340
   ccctagcgcc cgctcctttc gctttcttcc cttcctttct cgccacgttc gccggctttc 5400
   cccgtcaagc tctaaatcgg gggctccctt tagggttccg atttagtgct ttacggcacc 5460
   tcgaccccaa aaaacttgat tagggtgatg gttcacgtag tgggccatcg ccctgataga 5520
   cggtttttcg ccctttgacg ttggagtcca cgttctttaa tagtggactc ttgttccaaa 5580
   ctggaacaac actcaaccct atctcggtct attcttttga tttataaggg attttgccga 5640
   tttcggccta ttggttaaaa aatgagctga tttaacaaaa atttaacgcg aattttaaca 5700
   aaatattaac gcttacaatt tccattcgcc attcaggctg cgcaactgtt gggaagggcg 5760
   atcggtgcgg gcctcttcgc tattacgcca gctgatttaa atttaattaa ggcgcgccca 5820
   tggatcgatg ttaacatgca tgatatcacg cgtggcgcca ctagtgctag cagatctggc 5880
   cggcccaccg gtgggccata tgggcccgc 5909